# EUROPEAN PATENT SPECIFICATION

(11) **EP 0 752 895 B1**
(45) Date of publication and mention of the grant of the patent: **15.07.1998**
(21) Application number: 95913257.2
(22) Date of filing: 29.03.1995
(51) Int. Cl.: A61M 15/00

(54) **A STAND-ALONE COUNTER FOR A METERED DOSE INHALER**
ALLEINSTEHENDER ZÄHLER FÜR DOSIERINHALATOR
COMPTEUR ISOLE DESTINE A UN INHALATEUR DOSEUR

(30) Priority: 30.03.1994 GB 9406599
(43) Date of publication of application: 15.01.1997
(73) Proprietor: NORTON HEALTHCARE LIMITED, Harlow Essex CM19 5TJ (GB)
(72) Inventor: BACON, Raymond, Emworth Hampshire PO10 8QT (GB)
(74) Representative: Watkins, David
(86) International application number: GB9500725
(87) International publication number: WO9526769

(56) References cited:
- EP-A- 0 461 281
- WO-A-92/17231
- WO-A-93/24167
- US-A- 5 020 527
- US-A- 5 034 648

## Description

The present invention relates to a dose counter for a dispensing device of the type which is used for dispensing discrete amounts of a fluid or particulate material entrained in an air or other propellant stream. In particular, the invention is concerned with dose counters for dispensing devices of the metered dose inhaler type which are well known in the art of medicine for treatment of, or alleviation of the effects of, respiratory complaints such as asthma.

Metered dose inhalers typically consist of a medicament-containing vessel and an actuator body having a drug delivery outlet.

The medicament-containing vessel may be a pressurised canister containing a mixture of active drug and propellant. Such canisters are usually formed from a deep drawn aluminium cup portion having a crimped lid portion which carries a metering valve assembly. The metering valve assembly is provided with a protruding valve stem which, in use, is inserted as a tight push fit into a so-called "stem block" in the actuator body.

To actuate the "conventional" manually-operable inhaler, the user applies a compressive force to the closed end of the canister. The internal components of the metering valve assembly are spring loaded so that, typically, a compressive force of between 15 and 30 N is required to activate the device. In response to this compressive force, the canister moves axially with respect to the valve stem by an amount varying between about 2.0 and 3.5 mm. This degree of axial movement is sufficient to actuate the metering valve and cause a metered quantity of the drug and propellant to be expelled through the valve stem. This is then released into the mouthpiece *via* a nozzle in the stem block. A user inhaling through the drug delivery outlet of the device at this point will thus receive a dose of the drug.

Metered dose inhalers as described above administer an accurate dose of medicament whenever required, which is particularly useful for users whose respiratory difficulties manifest themselves suddenly. Such has been the success of these devices that they are now used throughout the world, where they are exposed to a wide variety of climatic conditions.

A more recent development is the so-called "breath-operated actuator" which delivers a dose of drug through a mouthpiece in response to inhalation by the user. This type of arrangement is particularly convenient in circumstances where the co-ordination between user inhalation and manual depression of the aerosol canister is imperfect. For example, children sometimes lack the necessary co-ordination to achieve effective self-administration. At times of respiratory distress, adult users may also experience poor co-ordination.

Unfortunately, one of the drawbacks of self-administration from an inhaler is that users often experience difficulty in determining when the charge in the medicament-containing vessel has nearly run out. With aerosol canisters, part of the reason for this difficulty is that a surplus of propellant may remain in the canister even though the drug supply is nearly used up. Alternatively, the near-exhausted state may result in a surplus of drug in relation to propellant. Thus, the illusion is created that the inhaler is still capable of providing useful doses of medicament simply because the canister contains liquid. This is potentially hazardous for the user since dosing becomes unreliable and because few people routinely carry a back-up device.

Many users have several different inhalers for the treatment of a variety of conditions. Others keep inhalers at a number of different locations such as at school, home, work etc. In these circumstances it is particularly difficult for the user to keep track of the amount of usage extracted from each individual inhaler apparatus.

Clearly there is a need for a counter mechanism which is capable of counting the number of doses delivered from or remaining in an inhaler. To this end, a number of counters have been proposed in recent times which aid the management of metered dosage. Such counters vary in complexity and sophistication, but they all have in common the feature that they detect relative movement between the medicament-containing vessel and the actuator body and increment in response to such movement.

One of the drawbacks of these known counters is that they rely on mechanical interaction between parts attached to the medicament-containing vessel and parts provided on the actuator body. This means that routine cleaning of the inhaler, which requires removal of the medicament-containing vessel from the actuator body, may result in damage to the counting mechanism. Also, the accumulated count may be corrupted by this operation.

Another drawback is that such counters are difficult to manufacture with satisfactory tolerances. A factor which influences the effectiveness of known displacement-triggered counters is the variation in length of typical aerosol canisters. This variation is attributable in part to the crimping operation used to connect the valve-carrying lid portion to the main cup portion. Although the technology involved is not especially demanding, it has been found that the amount of travel effective to actuate the metering valve of a typical medicament-containing aerosol canister may fall in a tolerance band as small as 0.5 mm. Thus it is difficult to provide a generic counter which increments accurately in response to every actuation. This may be true even when the counter, the aerosol canister and the inhaler housing have been specifically designed for use together. The problem is therefore likely to be worse in circumstances where different manufacturers' aerosol canisters, inhaler housings and displacement-triggered counters are used in combination.

The closest prior art is represented by document WO-A-9 217 231 which discloses a dose counter according to the preamble of claim 1. Other relevant prior art documents are US-A-5 020 527 and WO-A-9 324 167.

It is therefore an object of the present invention to provide a counter for a metered dose inhaler which does not rely on displacement triggering or the mechanical engagement between respective parts carried on the medicament-containing vessel and on the actuator housing.

It is a further object of the invention to provide a counter which allows removal of the medicament-containing vessel from the actuator body for routine cleaning without the risk of count corruption.

The invention is a stand-alone dose counter for a metered dose inhaler of the type comprising a medicament-containing vessel and an actuator body having a drug delivery outlet, said dose counter being provided with pressure sensing means, counting means having a count display and means to increment the count display in direct response to the application of a predetermined pressure upon the pressure sensing means, characterised in that said dose counter is provided with attachment means for attaching the dose counter to the base of a medicament-containing vessel.

One of the main advantages of a pressure-triggered counter compared to a displacement-triggered counter in the context of metered dose inhalers is that typical valve actuating pressures fall within a sensible range. This is in contrast to the valve actuating travel which, as mentioned above, may vary considerably from one inhaler to another. It is therefore relatively simple to provide a pressure-triggered device having a predetermined pressure threshold which renders it universally applicable. This is much easier to achieve in engineering terms than a displacement-triggered counter which is required to increment in response to a relative displacement as low as 0.5 mm.

It is preferred that the value of the predetermined pressure applied to the pressure sensing means which causes the count display to increment should be no greater than the compressive force required to actuate the metering valve of the aerosol canister. If this criterion is not met, the dose counter may under-count.

Errors biased in this direction, as opposed to over-counting, could have serious consequences for the user. This is because the implication that effective doses of medicament remain when the drug supply is actually exhausted could be potentially life-threatening.

Another important consideration is resistance to vibration. Often, the directions for use instruct the user to shake the dispenser prior to administration of the metered dose. The dose counter should therefore be able to withstand vigorous shaking without corruption of the recorded count. Similarly, the count should not be corrupted if the inhaler is accidentally dropped.

Preferably, the pressure sensing means is a solid state device, most preferably a piezo film sensor. In such an arrangement, the pressure sensing means generates an electrical output in proportion to the applied compressive force. The electrical output may then be fed into processing means such as a microprocessor in which the signal is analysed to determine whether the threshold of the predetermined minimum pressure has been reached. If so, an electrical impulse is sent to the counting means to effect incrementation of the count display.

Alternatively, the pressure sensing means may be a force-sensitive switch to which a predetermined force must be applied to overcome a biasing pressure that urges the switch contacts into an open (or closed) condition. When the switch contacts are closed (or opened), an electrical circuit is completed (or broken) which stimulus can be used to activate the counting means in order to increment the count display.

In an especially preferred form of the invention, the count display is provided on a surface of the dose counter which is visible from the exterior of the inhaler. This means that the display is visible to the user both before and after the administration of a dose of the drug. Such an arrangement is most conveniently implemented by means of an electronic chip with associated display function.

As indicated above, the dose counter is adapted to be attached to the closed end, i.e. the base, of an aerosol canister containing medicament and propellant under pressure. The dose counter may be in the form of a button and actuation of the inhaler is effected by manually depressing the button. This, in turn, moves the aerosol canister axially and actuates the metering valve. The force exerted on the button also causes incrementation of the counter. Conveniently, the counter display is provided on the actuating surface of the button.

Preferably, the button is provided with a resilient skirt for attaching it to the closed end of the aerosol canister in a reliable fashion. In order to make the dose counter fit a variety of aerosol canisters of differing diameters, the skirt may be provided with auxiliary adaptor means. Preferably the auxiliary adaptor means is easily removed and discarded if not required.

The button may incorporate a so-called "snap-dome" trigger which requires a consistent, minimum force to actuate. This type of arrangement aids the user in applying an undeviating activation pressure which ensures that the pressure sensing means is positively actuated whenever the trigger is fired. When the user relaxes the compressive force, the dome snaps back to its original position. Many users prefer the positive action which such a trigger imparts.

If desired, the pressure sensing means may be positioned at a remote location from other components of the counter, being connected thereto by trailing wires, for example. Such an arrangement allows for greater versatility in deployment of the counter and applicability to more varieties of inhaler.

Preferably, the dose counter is provided with a resetting mechanism operable to reset the count display when the counter is transferred from an exhausted inhaler apparatus. This may be implemented, for example, by means of a reset button which is adapted to make contact with a resetting circuit provided in the counter device. Once this contact is made, the count display is returned to its starting value. In order to avoid accidental resetting, it is preferable for the reset button to be concealed or recessed. For example, the reset button could be in the form of a recessed pin which is operable by means of an everyday object of narrow section, such as the tip of a ball-point pen.

The invention will now be described by way of example only with reference to the drawings, in which:
- Figure 1: shows a schematic cross-sectional view of a metered dose inhaler apparatus fitted with a dose counter in accordance with the invention, and
- Figure 2: is an exploded view showing the components of a dose counter according to the invention.

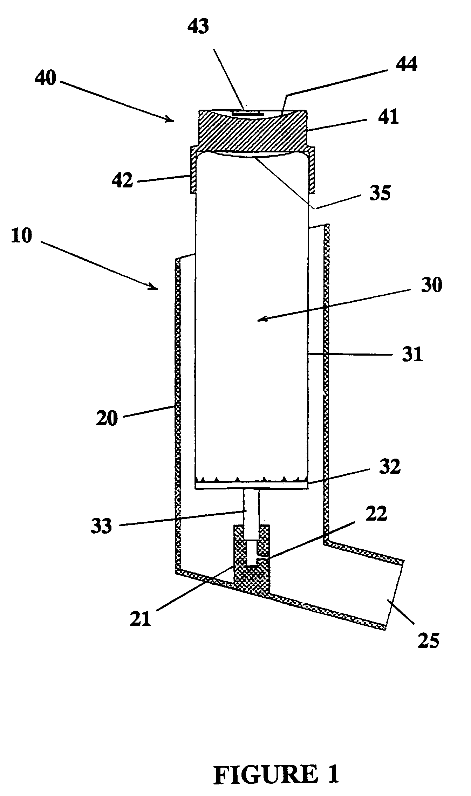

Referring now to Figure 1, reference numeral 10 denotes a metered dose inhaler comprising an actuator body 20 having a drug delivery outlet 25, and an aerosol canister 30. The aerosol canister 30 is formed from a deep drawn aluminium cup section 31 to which a lid portion 32 is attached by crimping. The lid portion 32 carries a metering valve assembly having a protruding valve stem 33, the end of which is received as a tight push fit in a stem block 21 of the actuator body 20. Stem block 21 has a nozzle 22 communicating with the drug delivery outlet 25 so that, upon actuation of the metering valve assembly, a charge of the drug is emitted through the nozzle 22 into the drug delivery outlet 25. A user inhaling through the drug delivery outlet 25 at this time will receive a metered dose of the drug.

The closed end 35 of aerosol canister 30 is provided with a dose counter 40 which comprises a body portion 41 and a depending skirt 42. The skirt 42 is formed of a resilient material which grips the outside of the aerosol canister 30 to maintain the dose counter 40 in position. Body portion 41 is in the form of a button which includes pressure sensing means and which has, on its top surface 44, a count display 43. Body portion 41 includes a processing chip and associated electronics (not shown) which are configured to determine whether a predetermined force has been applied to the pressure sensing means. When this force threshold is reached, the chip sends an electrical impulse to the count display 43 in order to effect incrementation thereof.

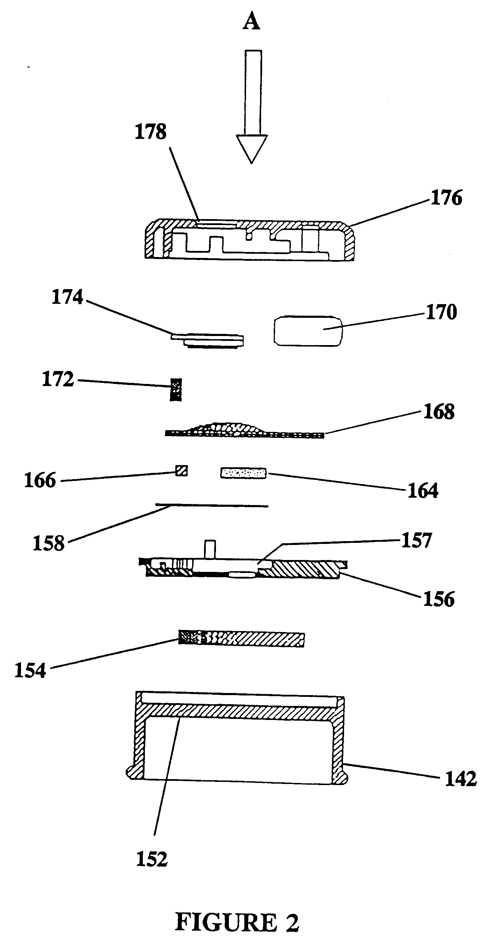

Turning now to Figure 2, the dose counter 40 of Figure 1 will be described in more detail with reference to the exploded view of its components.

A skirt element 142 is integrally moulded with a seat portion 152 on which a first resilient member 154 is placed. An intermediate housing 156 is adapted for placement on first resilient member 154, the upper surface of housing 156 being configured to receive a piezo film element 158 in a well 157. Overlying the piezo film element 158 is a second resilient member and a conducting element 166 which conveys electrical impulses from the piezo film element 158 to integrated circuit 168. Integrated circuit 168 is powered by a battery 170 which activates a liquid crystal counter display 174 through the integrated circuit 168 via connector 172. The assembly is completed by an outer cover 176 having a window 178 through which the counter display 174 is visible. The respective housing parts 152, 156 and 176 may be held together by bolts or other suitable fastening means.

In use, the fully-assembled device is installed on an aerosol canister as shown in Figure 1, with substantially no dead space between the closed end of the canister and the underside of the seat portion 152. Then, application of pressure to outer cover 176 in the direction of arrow A causes deflection of the piezo film element 158 inside the assembly at the same time as the canister is caused to translate within its holder. Deflection of the piezo film element 158 generates an electrical impulse which is transmitted to the integrated circuit 168.

A piezo film such as the element 158 is a force-sensitive device, so the degree of deflection will determine the strength of the generated electrical impulse. If the condition is satisfied that the electrical impulse exceeds a minimum threshold value, the count display 174 is caused to increment.

Ideally, the threshold value is set at a level which ensures that the dose counter does not under-count. In other words, the activation threshold of the counter should be equal to or less than the activation threshold of the metering valve assembly of the aerosol canister to which it is attached in use.

When the applied pressure is removed, the piezo film element 158 relaxes to a neutral position, aided by first and second resilient elements 154, 164.

Although the invention has been particularly described above in relation to certain specific embodiments, it will be understood by persons skilled in the art that its application is not limited to inhalers which use pressurised aerosol dispensers. For example, the invention may be applied to dry powder inhalers provided that their mechanism of operation is such that pressure sensing is a legitimate parameter to equate with dose counting. Other variations and modifications are possible and will be apparent to skilled persons without departing from the scope of the claims which follow.

## Claims

1. A stand-alone dose counter (40) suitable for use with a metered dose inhaler (10) of the type comprising a medicament-containing vessel (30) and an actuator body (20) having a drug delivery outlet (25), said dose counter (40) being provided with pressure sensing means (154, 158, 164), counting means having a count display (43, 174) and means to increment the count display (43, 174) in direct response to the application of a predetermined pressure upon the pressure sensing means (154, 158, 164), characterised in that said dose counter (40) is provided with attachment means (42) for attaching the dose counter (40) to the base (35) of a medicament-containing vessel (30).

2. A stand-alone dose counter (40) as claimed in claim 1, wherein the dose counter (40) is configured as button means (41).

3. A stand-alone dose counter (40) as claimed in claim 2, wherein said attachment means comprises a resilient skirt (42) depending from the button means (41) for attaching the button means (41) to the base of said medicament-containing vessel (30).

4. A stand-alone dose counter (40) as claimed in any preceding claim in which the pressure sensing means (154, 158, 164) is a solid state device such as a piezo film sensor (158).

5. A stand-alone dose counter (40) as claimed in any preceding claim wherein the pressure sensing means (154, 158, 164) is a force-sensitive switch the contacts of which are held open by a biasing force corresponding to said predetermined pressure.

6. A stand-alone dose counter (40) as claimed in any preceding claim in which a microprocessor (168) is used to determine whether the threshold of the minimum predetermined pressure has been reached.

7. A stand-alone dose counter (40) as claimed in any preceding claim wherein the count display (43, 174) is provided on a surface of the dose counter (40) which is visible from the exterior of the inhaler (10).

8. A stand-alone dose counter (40) as claimed in any preceding claim having resetting means operable to reset the count when the counter is transferred to a new inhaler apparatus.

9. A stand-alone dose counter (40) as claimed in claim 8 wherein the resetting means is recessed to prevent accidental actuation thereof during normal use.

## Patentansprüche

1. Ein alleinstehender Dosierzähler (40), geeignet zur Benutzung zusammen mit einem Inhalator (10) für eine abgemessene Dosis des Typs, der ein Gefäß (30) zur Aufnahme eines Medikaments und einen Betätigungskörper (20) mit einem Wirkstoffabgabeauslaß (25) aufweist, wobei der Dosierzähler (40) mit Druckerfassungseinrichtungen (154, 158, 164) versehen ist, sowie mit Zähleinrichtungen mit einer Zählanzeige (43, 174) und Einrichtungen zur Erhöhung der Zählanzeige (43, 174) in direkter Ansprache auf die Anwendung eines vorbestimmten Drucks auf die Druckerfassungseinrichtungen (154, 158, 164), dadurch gekennzeichnet, daß der Dosierzähler (40) mit Befestigungseinrichtungen (42) vorgesehen ist, um den Dosierzähler (40) an der Basis (35) eines Behälters (30) zur Aufnahme eines Medikaments anzubringen.

2. Ein alleinstehender Dosierzähler (40) nach Anspruch 1, bei dem der Dosierzähler (40) als eine Druckknopfvorrichtung (41) ausgebildet ist.

3. Ein alleinstehender Dosierzähler (40) nach Anspruch 2, bei dem die Anbringungsvorrichtungen eine federnde Schürze (42) umfaßt, die sich von der Druckknopfvorrichtung (41) erstreckt, um die Druckknopfvorrichtung (41) an der Basis des Behälters (30) zur Aufnahme des Medikaments zu befestigen.

4. Ein alleinstehender Dosierzähler (40) gemäß einem der vorstehenden Ansprüche, bei dem die Druckerfassungseinrichtungen (154, 158, 164) eine Festkörpereinrichtung etwa wie ein Piezo-Filmsensor (158) ist.

5. Ein alleinstehender Dosierzähler (40) nach einem der vorstehenden Ansprüche, bei dem die Druckerfassungseinrichtungen (154, 158, 164) ein auf Kräfte ansprechender Schalter sind, dessen Kontakte durch eine Vorspannungskraft offengehalten werden, welche dem vorbestimmten Druck entspricht.

6. Ein alleinstehender Dosierzähler (40) nach einem der vorstehenden Ansprüche, in den ein Mikroprozessor (168) dafür verwendet wird, festzustellen, ob die Schwelle des geringsten voreingestellten Drucks erreicht ist.

7. Ein alleinstehender Dosierzähler (40) nach einem der vorstehenden Ansprüche, bei dem die Zählanzeige (43, 174) auf einer Oberfläche des Dosierzählers (40) angeordnet ist, welche am Äußeren des Inhalators (10) sichtbar ist.

8. Ein alleinstehender Dosierzähler (40) nach einem der vorstehenden Ansprüche mit Rückstelleinrichtungen, die zur Zurückstellung der Zählung betätigbar sind, wenn der Zähler mit einem neuen Inhalatorgerät verbunden wird.

9. Ein alleinstehender Dosierzähler (40) nach Anspruch 8, bei welchem die Rückstelleinrichtung zurückgesetzt ist, um ihre versehentliche Betätigung bei normalem Gebrauch zu verhindern.

## Revendications

1. Une compteur de dose indépendant (40) adapté pour l'utilisation avec un inhalateur doseur (10) du type comprenant un récipient de médicament (30) et un corps d'actionnement (20) doté d'un orifice de sortie de délivrance du produit pharmaceutique (25), ledit compteur de dose (40) étant muni de moyens de détection de pression (154, 158, 164), des moyens de comptage munis d'un afficheur de comptage (43, 174) et des moyens pour incrémenter l'afficheur de comptage (43, 174) directement en réponse à l'application d'une pression prédéterminée sur les moyens de détection de pression (154, 158, 164), caractérisé en ce que le compteur de dose (40) est muni de moyens de fixation (42) pour fixer le compteur de dose (40) à la base (35) d'un récipient contenant un médicament (30).

2. Un compteur de dose indépendant (40) selon la revendication 1, dans lequel le compteur de dose (40) est réalisé sous la forme d'un bouton (41).

3. Un compteur de dose indépendant (40) selon la revendication 2, dans lequel les moyens de fixation comprennent une collerette élastique (42) dépendant du bouton (41) pour fixer le bouton (41) à la base dudit récipient contenant un médicament (30).

4. Un compteur de dose indépendant (40) selon l'une quelconque des revendications précédentes dans lequel les moyens de détection de pression (154, 158, 164) sont un élément à l'état solide tel qu'un capteur d'un film piézoélectrique (158).

5. Un compteur de dose indépendant (40) selon l'une quelconque des revendications précédentes, dans lequel les moyens de détection de pression (154, 158, 164) sont un interrupteur sensible à la force dont les contacts sont maintenus ouverts par une force de précharge correspondant à ladite pression prédéterminée.

6. Un compteur de dose indépendant (40) selon l'une quelconque des revendications précédentes, dans lequel un microprocesseur (168) est utilisé pour déterminer si le seuil de pression minimale prédéterminée à été atteint.

7. Un compteur de dose indépendant (40) selon l'une quelconque des revendications précédentes, dans lequel l'afficheur de comptage (43, 174) est prévu sur une surface d'un compteur de dose (40) qui est visible de l'extérieur de l'inhalateur (10).

8. Un compteur de dose indépendant (40) selon l'une quelconque des revendications précédentes, ayant des moyens de remise à zéro pouvant être actionnés pour remettre à zéro le compte lorsque le compteur est transféré à un nouvel appareil inhalateur.

9. Un compteur de dose indépendant (40) selon la revendication 8, dans lequel les moyens de remise à zéro sont encastrés pour empêcher l'actionnement accidentel de celui-ci durant l'utilisation normale.
